# EUROPEAN PATENT SPECIFICATION

(11) **EP 4 561 452 B1**
(45) Date of publication and mention of the grant of the patent: **01.07.2026**
(21) Application number: 23783196.1
(22) Date of filing: 13.09.2023
(51) Int. Cl.: A61B 8/08, A61B 8/12, A61B 8/00

(54) **GRAPHICAL USER INTERFACE FOR INTRAVASCULAR ULTRASOUND STENT DISPLAY**
GRAFISCHE BENUTZEROBERFLÄCHE FÜR INTRAVASKULÄRE ULTRASCHALLSTENTANZEIGE
INTERFACE GRAPHIQUE UTILISATEUR POUR AFFICHAGE D'ENDOPROTHÈSE INTRAVASCULAIRE À ULTRASONS

(30) Priority: 14.09.2022 US 202263406343 P
(43) Date of publication of application: 04.06.2025
(60) Divisional application: 26175430.3
(73) Proprietor: Boston Scientific Scimed Inc., Maple Grove, Minnesota 55311 (US)
(72) Inventor: WANG, Judith Tiferes, Buenos Aires (AR); GIBBS, Jennifer, White Bear, Minnesota 55110 (US)
(74) Representative: Vossius & Partner Patentanwälte Rechtsanwälte mbB
(86) International application number: PCT/US2023/032638
(87) International publication number: WO 2024/059136

(56) References cited:
- WO-A1-2014/092755
- US-A1- 2019 099 080
- US-A1- 2019 365 480

## Description

### TECHNICAL FIELD

The present disclosure generally relates to intravascular ultrasound (IVUS) imaging systems. Particularly, but not exclusively, the present disclosure relates to an improved graphical user interface for IVUS imaging systems

### BACKGROUND

Ultrasound devices insertable into patients have proven diagnostic capabilities for a variety of diseases and disorders. For example, intravascular ultrasound (IVUS) imaging systems have been used as an imaging modality for diagnosing blocked blood vessels and providing information to aid medical practitioners in selecting and placing stents and other devices to restore or increase blood flow.

IVUS imaging systems include a control module (with a pulse generator, an image acquisition and processing components, and a monitor), a catheter, and a transducer disposed in the catheter. The transducer-containing catheter is positioned in a lumen or cavity within, or in proximity to, a region to be imaged, such as a blood vessel wall or patient tissue in proximity to a blood vessel wall. The pulse generator in the control module generates electrical pulses that are delivered to the transducer and transformed to acoustic pulses that are transmitted through patient tissue. The patient tissue (or other structure) reflects the acoustic pulses and reflected pulses are absorbed by the transducer and transformed to electric pulses. The transformed electric pulses are delivered to the image acquisition and processing components and converted into images displayable on the monitor.

IVUS systems can be used to image a vessel after a stent is deployed to confirm correct placement of the stent. Thus, there here is a need for user interfaces, and particularly graphical user interfaces, that communicate information from the IVUS system to a user related to the deployment of the stent. US 2019/365480 A1 discloses a method which generates a first representation of a stented segment of the blood vessel indicative of a level of stent expansion, determines using the detected stent struts, a first end of the stent and a second end of the stent, and generates a second representation of the segment of the blood vessel by interpolating a lumen profile using an offset distance from the first end and the second end. US 2019/099080 A1 discloses a method for processing an intravascular image including a plurality of image frames acquired during a pullback of an imaging catheter inserted into a vessel, the method including displaying on a graphical user interface (GUI) an image including detected results of lumen borders and at least one stent, the image including an evaluated stent expansion and an evaluated stent apposition determined from the intravascular image. The method also includes determining whether a modification to the detected results of the stent has been received by the GUI. WO 2014/092755 A1 discloses a method for sizing a stent for placement in a vessel.

### BRIEF SUMMARY

This Summary is provided to introduce a selection of concepts in a simplified form that are further described below in the Detailed Description. This Summary is not intended to necessarily identify key features or essential features of the claimed subject matter, nor is it intended as an aid in determining the scope of the claimed subject matter.

In general, the present disclosure provides to an improvement to computing devices and particularly to IVUS guidance systems in that the present disclosure provides a graphical user interface arranged to convey the wealth of information with which modern IVUS systems generate. For example, an IVUS system may include machine learning features to process and analyze the signals generated during an IVUS run. Such information can include automatic detection of a lesion, key frames related to a lesion, a stent, or the like. The improved graphical user interface provided herein includes displaying such information as well as providing a method for a user to manipulate the information as needed.

In some implementations, the present disclosure be embodied as a method, for example, a method for an intravascular ultrasound (IVUS) imaging system, comprising: receiving a series of intravascular ultrasound (IVUS) images of a vessel of a patient, the series of IVUS images comprising a plurality of frames; receiving an indication of a location of a stent in the vessel; generating a first graphical user interface (GUI) component comprising an indication of a cross-section view of the vessel captured in one of the plurality of frames; generating a second GUI component comprising indications of at least one menu option; generating a third GUI component comprising indications of at least one layout option; generating a fourth GUI component comprising indications of a longitudinal view of the vessel captured in the plurality of frames and the location of the stent relative to the vessel; generating a GUI comprising the first, second, third, and fourth GUI components, wherein the first GUI component is disposed between the second and third GUI components; and rendering the GUI for display on a display.

Alternatively, or additionally any of the embodiments of a method above can comprise: generating a proximal key frame based on a proximal end of the stent; generating a distal key frame based on a distal end of the stent; and generating the fourth GUI component further comprising an indication of the proximal key frame and the distal key frame.

Alternatively, or additionally in any of the embodiments of a method above, the fourth GUI component can comprise an indication of the vessel and an indication of a lumen.

Alternatively, or additionally in any of the embodiments of a method above, the fourth GUI component can comprise a mirrored reflection of the indication of the vessel and the lumen about a longitudinal axis.

Alternatively, or additionally in any of the embodiments of a method above the fourth GUI component can comprise a colored, shaded, or patterned area between the lumen and the mirrored reflection of the lumen and the proximal key frame and the distal key frame to indicate the location of the stent relative to the vessel.

Alternatively, or additionally any of the embodiments of a method above can comprise determining an expansion of the stent, wherein the fourth GUI component comprises an indication of the expansion of the stent.

Additionally, the method above comprises determining a distal expansion of the stent and a proximal expansion of the stent, wherein the fourth GUI component comprises an indication of the distal expansion of the stent and an indication of the proximal expansion of the stent.

Alternatively, or additionally any of the embodiments of a method above can comprise: determining the distal expansion of the stent based on a minimum stent area (MSA) divided by a lumen area for areas distal of a minimum key frame; and determining the proximal expansion of the stent based on the minimum stent area (MSA) divided by the lumen area for areas proximal of the minimum key frame.

Alternatively, or additionally in any of the embodiments of a method above the distal expansion of the stent and the proximal expansion of the stent can be represented as percent, wherein the indication of the distal expansion of the stent comprises a line between the minimum frame marker and a distal end of the stent and a graphical indication of the percent of distal expansion, and wherein the indication of the proximal expansion of the stent comprises a line between the minimum frame marker and a proximal end of the stent and a graphical indication of the percent of proximal expansion.

Alternatively, or additionally any of the embodiments of a method above can comprise receiving, via an input device, an indication to move the proximal key frame, the distal key frame, or the proximal key frame and the distal key frame.

Alternatively, or additionally any of the embodiments of a method above can comprise determining an updated distal expansion of the stent and/or an updated proximal expansion of the stent based on the moved proximal key frame and/or the moved distal key frame.

Alternatively, or additionally any of the embodiments of a method above can comprise regenerating the fourth GUI component and the GUI to include an indication of the updated distal expansion of the stent and/or the updated proximal expansion of the stent.

Alternatively, or additionally in any of the embodiments of a method above the regenerated fourth GUI component can comprise the colored, shaded, or patterned area between the lumen and the mirrored reflection of the lumen and the moved proximal key frame and/or the moved distal key frame to indicate the updated location of the stent relative to the vessel.

In some implementations, the present disclosure be embodied as an apparatus, comprising a processor coupled to a memory, the memory comprising instructions executable by the processor, the processor configured to couple to an intravascular ultrasound (IVUS) imaging system and configured to execute the instructions, which instructions when executed cause the processor to implement the method of any combination of the examples above.

In some implementations, the present disclosure can be embodied as at least one machine readable storage device, comprising a plurality of instructions that in response to being executed by a processor of an intravascular ultrasound (IVUS) imaging system cause the processor to implement the method of any combination of the examples above.

In some implementations, the present disclosure be embodied as an apparatus for an intravascular ultrasound (IVUS) imaging system, comprising: a display; an interface configured to couple to an IVUS catheter; a processor coupled to the interface and the display; and a memory device comprising instruction, which when executed by the processor cause the IVUS imaging system to: receive a series of IVUS images of a vessel of a patient, the series of IVUS images comprising a plurality of frames; receive an indication of a location of a stent in the vessel; generate a first graphical user interface (GUI) component comprising an indication of a cross-section view of the vessel captured in one of the plurality of frames; generate a second GUI component comprising indications of at least one menu option; generate a third GUI component comprising indications of at least one layout option; generate a fourth GUI component comprising indications of a longitudinal view of the vessel captured in the plurality of frames and the location of the stent relative to the vessel; generate a GUI comprising the first, second, third, and fourth GUI components, wherein the first GUI component is disposed between the second and third GUI components; and render the GUI and send the rendered GUI to the display.

Alternatively, or additionally in any of the embodiments of an apparatus above, the memory device can further comprise instructions that when executed by the processor cause the IVUS imaging system to: generate a proximal key frame based on a proximal end of the stent; generate a distal key frame based on a distal end of the stent; and generate the fourth GUI component further comprising an indication of the proximal key frame and the distal key frame.

Alternatively, or additionally in any of the embodiments of an apparatus above, the fourth GUI component can comprise an indication of the vessel and an indication of a lumen.

Alternatively, or additionally in any of the embodiments of an apparatus above, the fourth GUI component can comprise a mirrored reflection of the indication of the vessel and the lumen about a longitudinal axis.

Alternatively, or additionally in any of the embodiments of an apparatus above, the fourth GUI component can comprise a colored, shaded, or patterned area between the lumen and the mirrored reflection of the lumen and the proximal key frame and the distal key frame to indicate the location of the stent relative to the vessel.

Alternatively, or additionally in any of the embodiments of an apparatus above, the memory device can further comprise instructions that when executed by the processor cause the IVUS imaging system to determine an expansion of the stent, wherein the fourth GUI component comprises an indication of the expansion of the stent.

Alternatively, or additionally in any of the embodiments of an apparatus above, the memory device can further comprise instructions that when executed by the processor cause the IVUS imaging system to determine a distal expansion of the stent and a proximal expansion of the stent, wherein the fourth GUI component comprises an indication of the distal expansion of the stent and an indication of the proximal expansion of the stent.

Alternatively, or additionally in any of the embodiments of an apparatus above, the memory device can further comprise instructions that when executed by the processor cause the IVUS imaging system to: determine the distal expansion of the stent based on a minimum stent area (MSA) divided by a lumen area for areas distal of a minimum key frame; and determine the proximal expansion of the stent based on the minimum stent area (MSA) divided by the lumen area for areas proximal of the minimum key frame.

Alternatively, or additionally in any of the embodiments of an apparatus above, the distal expansion of the stent and the proximal expansion of the stent are represented as percent, wherein the indication of the distal expansion of the stent comprises a line between the minimum frame marker and a distal end of the stent and a graphical indication of the percent of distal expansion, and wherein the indication of the proximal expansion of the stent comprises a line between the minimum frame marker and a proximal end of the stent and a graphical indication of the percent of proximal expansion.

Alternatively, or additionally in any of the embodiments of an apparatus above, the memory device can further comprise instructions that when executed by the processor cause the IVUS imaging system to receive, via an input device, an indication to move the proximal key frame, the distal key frame, or the proximal key frame and the distal key frame.

In some implementations, the present disclosure be embodied as at least one machine readable storage device, comprising a plurality of instructions that in response to being executed by a processor of an intravascular ultrasound (IVUS) imaging system cause the processor to: receive a series of IVUS images of a vessel of a patient, the series of IVUS images comprising a plurality of frames; receive an indication of a location of a stent in the vessel; generate a first graphical user interface (GUI) component comprising an indication of a cross-section view of the vessel captured in one of the plurality of frames; generate a second GUI component comprising indications of at least one menu option; generate a third GUI component comprising indications of at least one layout option; generate a fourth GUI component comprising indications of a longitudinal view of the vessel captured in the plurality of frames and the location of the stent relative to the vessel; generate a GUI comprising the first, second, third. and fourth GUI components, wherein the first GUI component is disposed between the second and third GUI components; and render the GUI for display on a display.

Alternatively, or additionally in any of the embodiments of an at least one machine readable storage device above, the instructions in response to being executed by the processor can further cause the processor to: generate a proximal key frame based on a proximal end of the stent; generate a distal key frame based on a distal end of the stent; and generate the fourth GUI component further comprising an indication of the proximal key frame and the distal key frame, wherein the fourth GUI component comprises an indication of the vessel and an indication of a lumen, and wherein the fourth GUI component comprises a mirrored reflection of the indication of the vessel and the lumen about a longitudinal axis.

Alternatively, or additionally in any of the embodiments of an at least one machine readable storage device above, the instructions in response to being executed by the processor can further cause the processor to: determine an expansion of the stent, wherein the fourth GUI component comprises an indication of the expansion of the stent; and determine a distal expansion of the stent and a proximal expansion of the stent, wherein the fourth GUI component comprises an indication of the distal expansion of the stent and an indication of the proximal expansion of the stent.

Alternatively, or additionally in any of the embodiments of an at least one machine readable storage device above, the instructions in response to being executed by the processor can further cause the processor to: determine the distal expansion of the stent based on a minimum stent area (MSA) divided by a lumen area for areas distal of a minimum key frame; and determine the proximal expansion of the stent based on the minimum stent area (MSA) divided by the lumen area for areas proximal of the minimum key frame.

Alternatively, or additionally in any of the embodiments of an at least one machine readable storage device above, the instructions in response to being executed by the processor can further cause the processor to: receive, via an input device, an indication to move the proximal key frame, the distal key frame, or the proximal key frame and the distal key frame; determine an updated distal expansion of the stent and/or an updated proximal expansion of the stent based on the moved proximal key frame and/or the moved distal key frame; and determine an updated distal expansion of the stent and/or an updated proximal expansion of the stent based on the moved proximal key frame and/or the moved distal key frame.

Alternatively, or additionally in any of the embodiments of an at least one machine readable storage device above, the instructions in response to being executed by the processor can further cause the processor to regenerate the fourth GUI component and the GUI to include an indication of the updated distal expansion of the stent and/or the updated proximal expansion of the stent.

### BRIEF DESCRIPTION OF THE SEVERAL VIEWS OF THE DRAWINGS

To easily identify the discussion of any element or act, the most significant digit or digits in a reference number refer to the figure number in which that element is first introduced.
FIG. 1 illustrates an IVUS imaging system.
FIG. 2 illustrates an angiographic image of a vessel.
FIG. 3A illustrates a longitudinal view of IVUS images.
FIG. 3B illustrates a cross-section view of a frame of IVUS images.
FIG. 4 illustrates IVUS images visualization system 400.
FIG. 5A illustrates a first graphical interface for an IVUS imaging system.
FIG. 5B illustrates a second graphical interface for an IVUS imaging system.
FIG. 6 illustrates a third graphical interface for an IVUS imaging system.
FIG. 7 illustrates a first graphical component of a graphical interface for an IVUS imaging system.
FIG. 8A illustrates a second graphical component of a graphical interface for an IVUS imaging system.
FIG. 8B illustrates a third graphical component of a graphical interface for an IVUS imaging system.
FIG. 9 illustrates a logic flow for generating a graphical interface for an IVUS imaging system.
FIG. 10 illustrates a computer-readable storage medium.
FIG. 11 illustrates a diagrammatic representation of a machine.

### DETAILED DESCRIPTION

The foregoing has broadly outlined the features and technical advantages of the present disclosure such that the following detailed description of the disclosure may be better understood. It is to be appreciated by those skilled in the art that the embodiments disclosed may be readily utilized as a basis for modifying or designing other structures for carrying out the same purposes of the present disclosure. The novel features of the disclosure, both as to its organization and operation, together with further objects and advantages will be better understood from the following description when considered in connection with the accompanying figures. It is to be expressly understood, however, that each of the figures is provided for the purpose of illustration and description and is not intended as a definition of the limits of the present disclosure.

As noted, the present disclosure relates to IVUS systems and automatic assessment of the IVUS images. In particular, the disclosure provides a graphical user interface (GUI) arranged to convey information related to the IVUS images and lesion assessment and provide for the user to manipulate the information. As such, an example IVUS imaging system, patient vessel, and series of IVUS images are described.

Suitable IVUS imaging systems include, but are not limited to, one or more transducers disposed on a distal end of a catheter configured and arranged for percutaneous insertion into a patient. Examples of IVUS imaging systems with catheters are found in, for example, U.S. Pat. Numbers 7,246,959; 7,306,561; and 6,945,938; as well as U.S. Patent Application Publication Numbers 2006/0100522; 2006/0106320; 2006/0173350; 2006/0253028; 2007/0016054; and 2007/0038111.

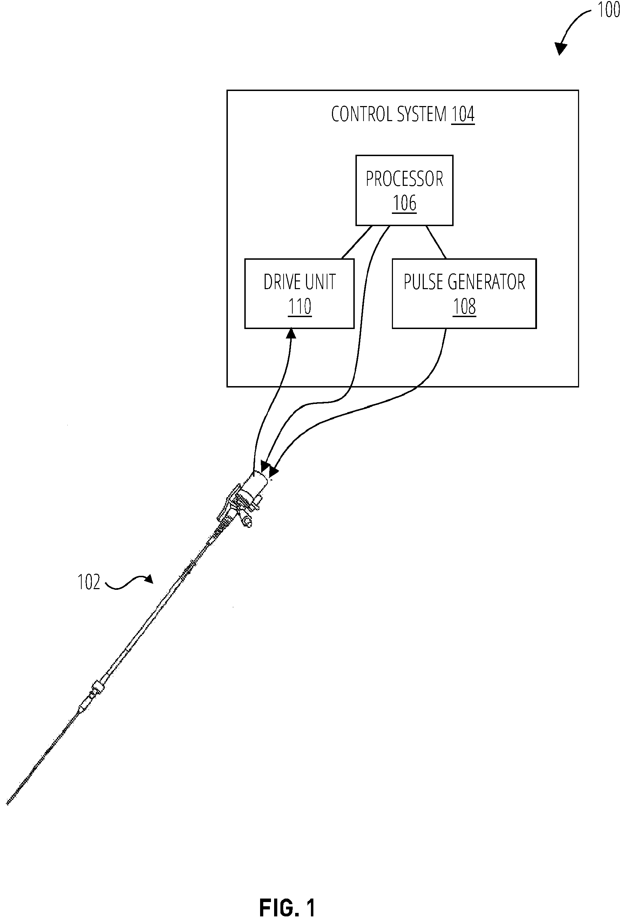

FIG. 1 illustrates one embodiment of an IVUS imaging system 100. The IVUS imaging system 100 includes a catheter 102 that is couplable to a control system 104. The control system 104 may include, for example, a processor 106, a pulse generator 108, and a drive unit 110. The pulse generator 108 forms electric pulses that may be input to one or more transducers (not shown) disposed in the catheter 102.

With some embodiments, mechanical energy from the drive unit 110 can be used to drive an imaging core (also not shown) disposed in the catheter 102. In at least some embodiments, electric signals transmitted from the one or more transducers may be input to the processor 106 for processing. In at least some embodiments, the processed electric signals from the one or more transducers can be used to form a series of images, described in more detail below. For example, a scan converter can be used to map scan line samples (e.g., radial scan line samples, or the like) to a two-dimensional Cartesian grid, which can be used as the basis for a series of IVUS images that can be displayed for a user.

In at least some embodiments, the processor 106 may also be used to control the functioning of one or more of the other components of the control system 104. For example, the processor 106 may be used to control at least one of the frequency or duration of the electrical pulses transmitted from the pulse generator 108, the rotation rate of the imaging core by the drive unit 110. Additionally, where IVUS imaging system 100 is configured for automatic pullback, the drive unit 110 can control the velocity and/or length of the pullback.

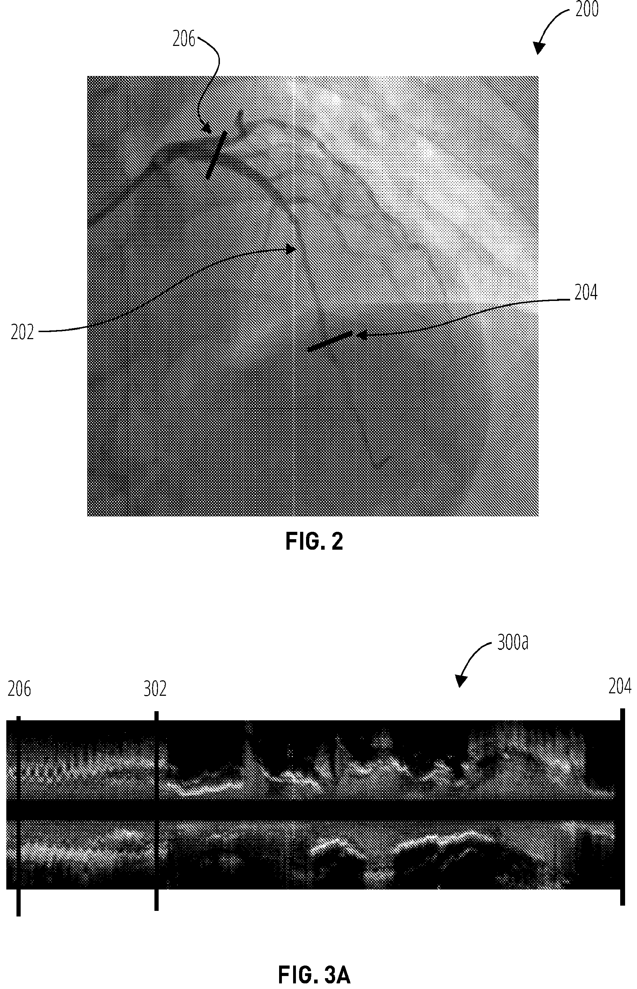

FIG. 2 illustrates an image 200 of a vessel 202 of a patient. As described, IVUS imaging systems (e.g., IVUS imaging system 100, or the like) are used to capture a series of images or a "recording" or a vessel, such as, vessel 202. For example, an IVUS catheter (e.g., catheter 102) is inserted into vessel 202 and a recording, or a series of IVUS images, is captured as the catheter 102 is pulled back from a distal end 204 to a proximal end 206. The catheter 102 can be pulled back manually or automatically (e.g., under control of drive unit 110, or the like).

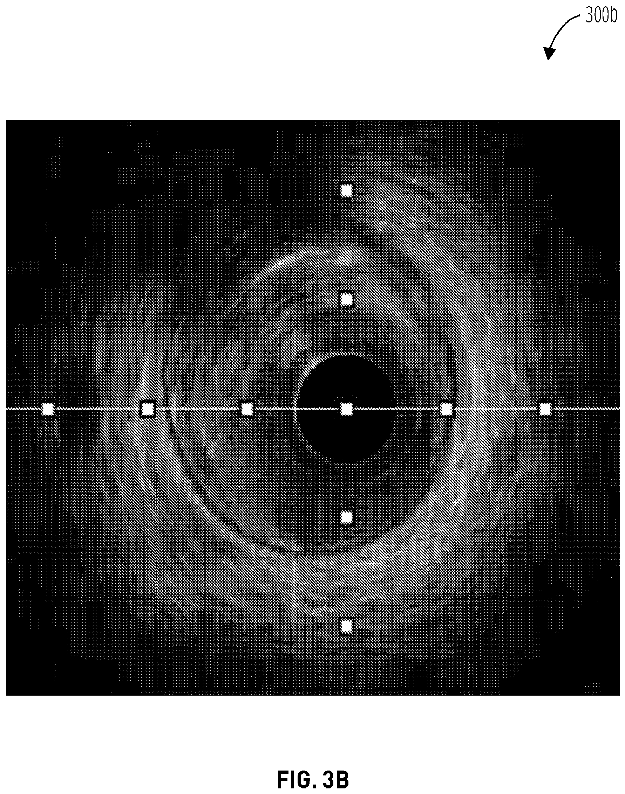

FIG. 3A and FIG. 3B illustrates two-dimensional (2D) representations of IVUS images of vessel 202. For example, FIG. 3A illustrates IVUS images 300a depicting a longitudinal view of the IVUS recording of vessel 202 between proximal end 206 and distal end 204.

FIG. 3B illustrates an image frame 300b depicting an on-axis (or short axis, or cross-section) view of vessel 202 at point 302. Said differently, image frame 300b is a single frame or single image from a series of IVUS images that can be captured between distal end 204 and proximal end 206 as described herein. As introduced above, the present disclosure provides systems and techniques to process raw IVUS images to identify regions of interest, such as, for example starting and ending points between which include frames of interest in a series of IVUS images.

For example, IVUS images 300a depicts an entire series of IVUS images taken of vessel 202 between distal end 204 and proximal end 206. IVUS images may be captured at several stages of a percutaneous coronary intervention (PCI). That is, IVUS may be employed pre-PCI, peri-PCI, or post-PCI. For example, IVUS may be employed to capture images of the state of the vessel 202 before a stent is implanted. In such an example, automatic assessments of the images can be performed (e.g., vessel border detection, lumen border detection, plaque burden detection, key frame identification, stent size and landing zone recommendations, stent expansion estimation, or the like). It is to be appreciated that this is a significant amount of information to convey to a user. Further, conveying this information along with the IVUS images (e.g., IVUS images 300a and 300b) in a manner that allows the user to manipulate the automatic assessments (e.g., key frames, or the like) is not provided in the prior art. Thus, the present disclosure provides an advantage in that the improved GUI provides to greater understanding and allows manipulation of the features of the image.

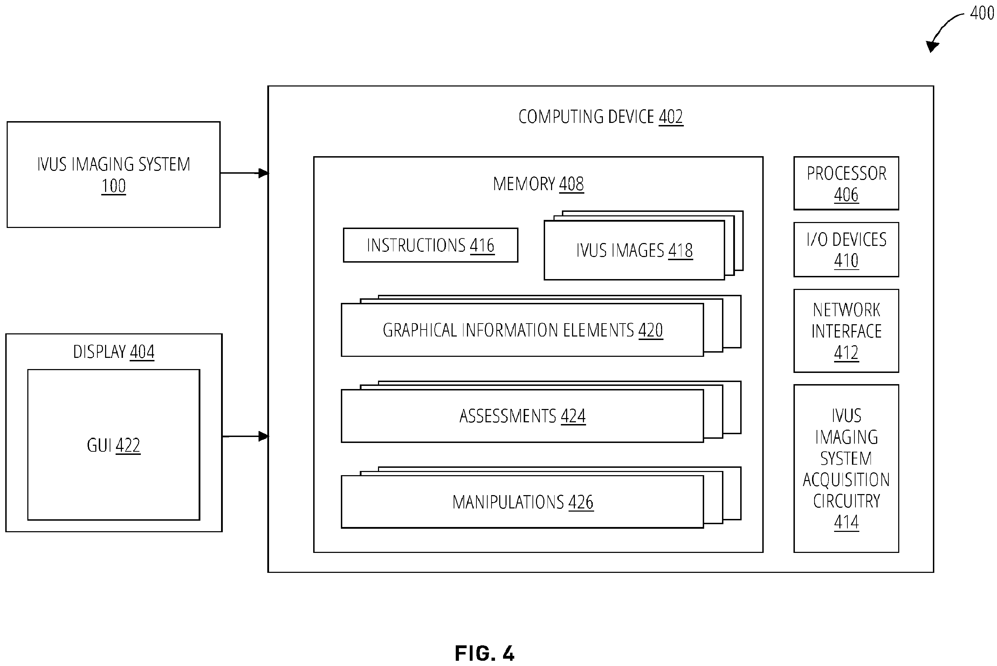

FIG. 4 illustrates an IVUS images visualization system 400, according to some embodiments of the present disclosure. In general, IVUS images visualization system 400 is a system for processing, annotating, and presenting IVUS images. IVUS images visualization system 400 can be implemented in a commercial IVUS guidance or navigation system, such as. for example, the AVVIGO ^{®} Guidance System available from Boston Scientific ^{®}. The present disclosure provides advantages over prior or conventional IVUS navigation systems in that the improved GUI will reduce the time needed for patients to be in treatment. For example, the present disclosure can be implemented in an IVUS navigation system to efficiently communicate IVUS information to a user and allow the user to manipulate the information.

With some embodiments, IVUS images visualization system 400 could be implemented as part of control system 104. Alternatively, control system 104 could be implemented as part of IVUS images visualization system 400. As depicted, IVUS images visualization system 400 includes a computing device 402. Optionally, IVUS images visualization system 400 includes IVUS imaging system 100 and display 404.

Computing device 402 can be any of a variety of computing devices. In some embodiments, computing device 402 can be incorporated into and/or implemented by a console of display 404. With some embodiments, computing device 402 can be a workstation or server communicatively coupled to IVUS imaging system 100 and/or display 404. With still other embodiments, computing device 402 can be provided by a cloud based computing device, such as, by a computing as a service system accessibly over a network (e.g., the Internet, an intranet, a wide area network, or the like). Computing device 402 can include processor 406, memory 408, input and/or output (I/O) devices 410, network interface 412, and IVUS imaging system acquisition circuitry 414.

The processor 406 may include circuity or processor logic, such as, for example, any of a variety of commercial processors. In some examples, processor 406 may include multiple processors, a multi-threaded processor, a multi-core processor (whether the multiple cores coexist on the same or separate dies), and/or a multi-processor architecture of some other variety by which multiple physically separate processors are in some way linked. Additionally, in some examples, the processor 406 may include graphics processing portions and may include dedicated memory, multiple-threaded processing and/or some other parallel processing capability. In some examples, the processor 406 may be an application specific integrated circuit (ASIC) or a field programmable integrated circuit (FPGA).

The memory 408 may include logic, a portion of which includes arrays of integrated circuits, forming non-volatile memory to persistently store data or a combination of non-volatile memory and volatile memory. It is to be appreciated, that the memory 408 may be based on any of a variety of technologies. In particular, the arrays of integrated circuits included in memory 120 may be arranged to form one or more types of memory, such as, for example, dynamic random access memory (DRAM), NAND memory, NOR memory, or the like.

I/O devices 410 can be any of a variety of devices to receive input and/or provide output. For example, I/O devices 410 can include, a keyboard, a mouse, a joystick, a foot pedal, a display, a touch enabled display, a haptic feedback device, an LED, or the like.

Network interface 412 can include logic and/or features to support a communication interface. For example, network interface 412 may include one or more interfaces that operate according to various communication protocols or standards to communicate over direct or network communication links. Direct communications may occur via use of communication protocols or standards described in one or more industry standards (including progenies and variants). For example, network interface 412 may facilitate communication over a bus, such as, for example, peripheral component interconnect express (PCIe), non-volatile memory express (NVMe), universal serial bus (USB), system management bus (SMBus), SAS (e.g., serial attached small computer system interface (SCSI)) interfaces, serial AT attachment (SATA) interfaces, or the like. Additionally, network interface 412 can include logic and/or features to enable communication over a variety of wired or wireless network standards (e.g., 1702.11 communication standards). For example, network interface 412 may be arranged to support wired communication protocols or standards, such as, Ethernet, or the like. As another example, network interface 412 may be arranged to support wireless communication protocols or standards, such as, for example, Wi-Fi, Bluetooth, ZigBee, LTE, 5G, or the like.

The IVUS imaging system acquisition circuitry 414 may include circuity including custom manufactured or specially programmed circuitry configured to receive or receive and send signals between IVUS imaging system 100 including indications of an IVUS run, a series of IVUS images, or a frame or frames of IVUS images.

Memory 408 can include instructions 416. During operation processor 406 can execute instructions 416 to cause computing device 402 to receive (e.g., from IVUS imaging system 100, or the like) a recording of an "IVUS run" and store the recording as IVUS images 418 in memory 408. For example, processor 406 can execute instructions 416 to receive information elements from IVUS imaging system 100 comprising indications of IVUS images captured by catheter 102 while being pulled back from distal end 204 to proximal end 206, which images comprising indications of the anatomy and/or structure of vessel 202 including vessel walls and plaque. It is to be appreciated that IVUS images 418 can be stored in a variety of image formats or even non-image formats or data structures that comprise indications of vessel 202. Further, IVUS images 418 includes several "frames" or individual images that, when represented co-linearly can be used to form an image of the vessel 202, such as, for example, as represented by IVUS images 300a and/or 300b.

The present disclosure provides to generate graphical information elements 420 from IVUS images 418 and to generate a GUI 422 to be displayed on display 404 based on the graphical information elements 420. Processor 406 can further be configured to execute instructions 416 to generate assessments 424 based on IVUS images 418. This will be described in greater detail below. However, in general, the assessments can include vessel boundary detection, lumen boundary detection, plaque burden determination, key frame identification, distances between key frames, stent detection, stent expansion estimates, among other assessments. As such, in some embodiments, graphical information elements 420 can be generated based on IVUS images 418 and assessments 424.

Additionally, processor 406 can be configured to execute instructions 416 to receive manipulations 426 including modifications to assessments 424. For example, processor 406 can execute instructions 416 to receive modifications to key frame locations, or the like and store such modifications as manipulations 426. Responsive to receiving manipulations 426, processor 406 can execute instructions 416 to regenerate graphical information elements 420 based on IVUS images 418, assessments 424, and/or manipulations 426. For example, processor 406 can execute instructions 416 to redetermine the stent expansion percentages based on the manipulations 426 and regenerate graphical information elements 420 to corresponds to the updated stent expansion percentages.

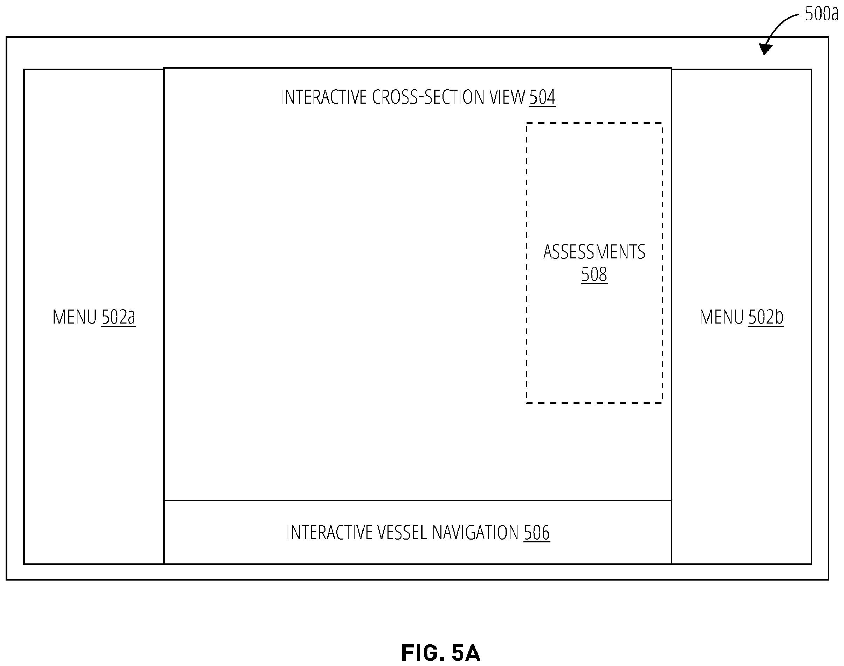

FIG. 5A illustrates a GUI 500a, which can be generated according to some embodiments of the present disclosure. For example, GUI 500a can be generated by IVUS images visualization system 400 as GUI 422 and displayed on display 404. As depicted, GUI 500a includes several graphical information elements 420, such as, menus 502a and 502b, interactive cross-section view 504, and interactive vessel navigation 506. With some embodiments, processor 406 can be configured to execute instructions 416 to generate GUI 500a once assessment activation button 506 is selected. As another example, processor 406 can execute instructions 416 to generate GUI 500a responsive to an automatic stent detection process. For example, with some embodiments, IVUS images visualization system 400 can be arranged to automatically detect stents from IVUS images 418 (e.g., via machine learning, image classification, or the like). Responsive to detection of a stent in IVUS images 418, key frames can be determined based on the location of the detected stent and GUI 500a can be generated.

Menu 502a can comprise GUI inputs such as button, drop down menus, selection icons, or the like. Menu 502a can include GUI input options to select measurement and annotation tools, length tools, modification reset buttons, or the like. Menu 502b can comprise GUI inputs such as buttons, drop down menus, selection icons, or the like. Menu 502b can include GUI inputs options to select views related to views of the IVUS images, layout options, annotations, navigation, dynamic review options, status of the computing device, or the like.

Interactive cross-section view 504 can comprise a cross-sectional view of a one (e.g., a frame, or the like) of IVUS images 418. For example, interactive cross-section view 504 can include image frame 300b and assessments 508 as well as indications of vessel and lumen borders. A detailed description of interactive vessel navigation 506 is provided below. However, in general, interactive vessel navigation 506 can include a navigation slider to navigation through IVUS images 418, which is linked to the interactive cross-section view 504. That is, as the slider is moved the image displayed in interactive cross-section view 504 changes to match the location indicated by the slider. Further interactive vessel navigation 506 can include representations of a vessel and lumen profile as well as an indication of a stent, key frames, a minimum region, and stent expansion. For example, the stent location can be indicated with a different pattern of color from other portions of the GUI 500a. Additionally, the location of distal and proximal frames (e.g., distal, and proximal key frames) linearly along the series of IVUS images 418 can be indicated (c.g., with brackets, or the like).

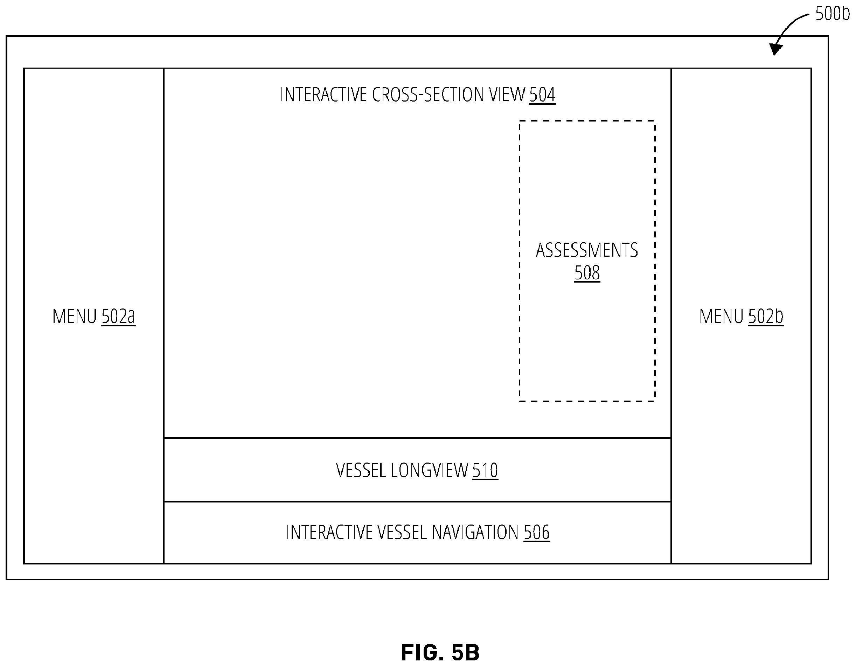

FIG. 5B illustrates a GUI 500b, which can be generated according to some embodiments of the present disclosure. For example, GUI 500b can be generated by IVUS images visualization system 400 as GUI 422 and displayed on display 404. As depicted, GUI 500b includes several graphical information elements 420 like GUI 500a. Notably, GUI 500b further includes vessel long view 510. Vessel long view 510 can comprise a longitudinal view of the vessel (e.g., vessel 202) represented by the IVUS images 418. For example, vessel long view 510 can include IVUS images 300a.

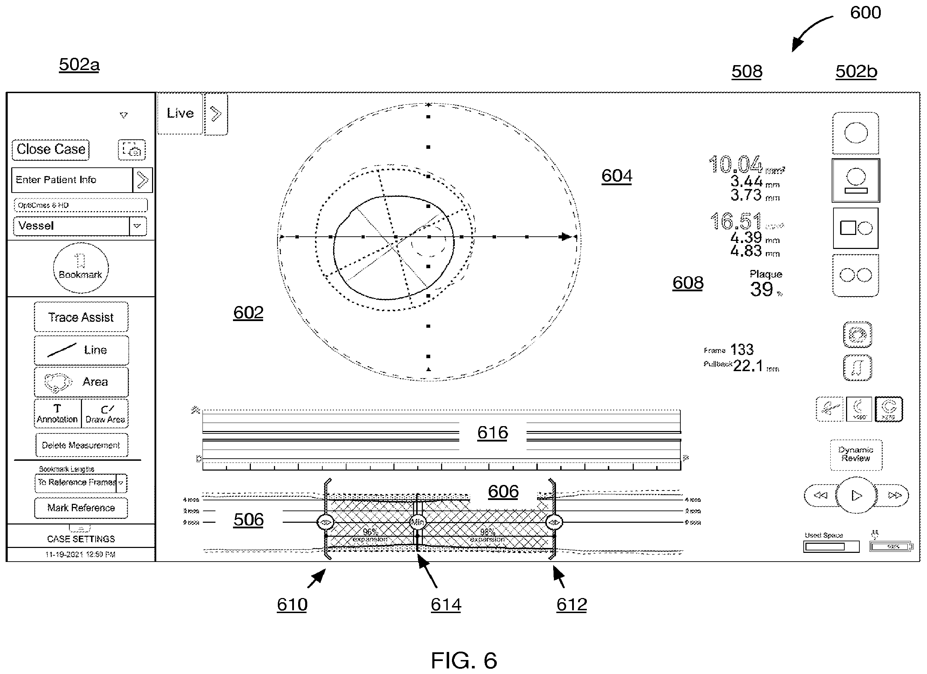

FIG. 6 illustrates a GUI 600, which can be generated according to some embodiments of the present disclosure. For example, GUI 600 can be generated by IVUS images visualization system 400 as GUI 422 and displayed on display 404. With some embodiments, responsive to detection of a stent, processor 406 can execute instructions 416 to generate graphical information elements 420 and GUI 600 from graphical information elements 420.

As depicted, GUI 600 includes menu 502a and menu 502b disposed on either sides of (or framing) interactive cross-section view 504 and interactive vessel navigation 506. As depicted interactive cross-section view 504 includes depictions or representations of a cross-section view 602 (e.g., corresponding to the point in IVUS images 418 at which view slider 608 is disposed) as well as depictions or representations of borders 604 (e.g., lumen border, vessel border, diameters, etc.) and assessments 508.

Additionally, interactive vessel navigation 506 includes distal bracket end 610, proximal bracket end 612, slider axis 616, view slider 608, profile view 606, and minimum region 614. Examples of profile view 606 and minimum region 614 are given below.

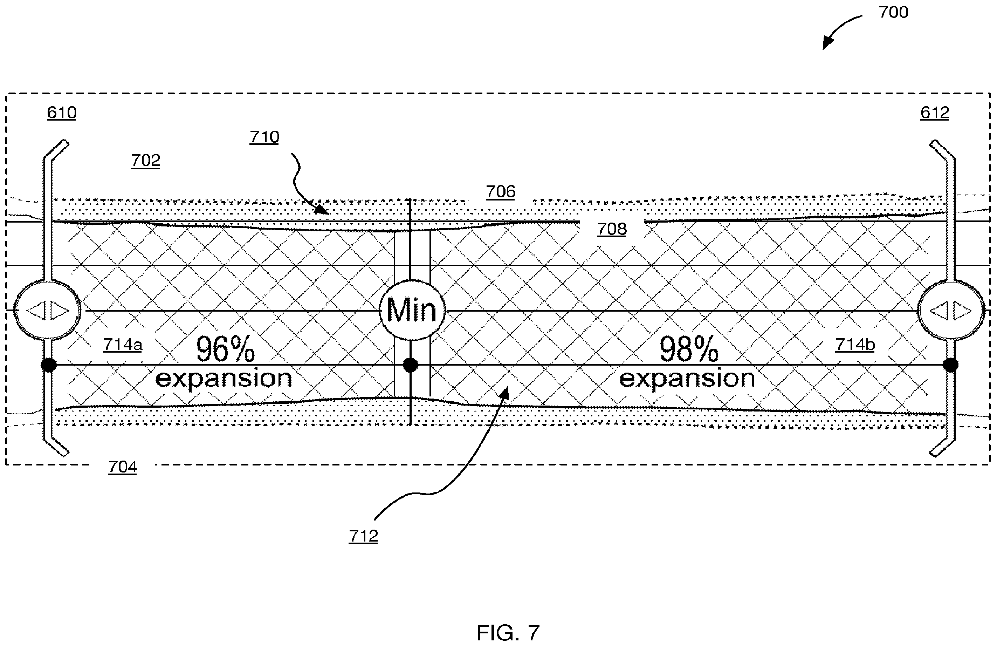

FIG. 7 illustrates a profile view 700, which can be generated according to some embodiments of the present disclosure. For example, profile view 700 can be generated by IVUS images visualization system 400 as interactive vessel navigation 506 of GUI 422 and displayed on display 404. As depicted, profile view 700 can include longitudinal border profile 704 and longitudinal border profile mirror reflection 708, which each include vessel border 702 and lumen border 706. For example, as outlined above, with some examples, processor 406 can execute instructions 416 to automatically detect the vessel and lumen borders. Further, processor 406 can execute instructions 416 to represent the detected vessel and lumen borders as vessel border 702 and lumen border 706. Further still, processor 406 can execute instructions 416 to mirror a graphical representation of the detected borders to present a more realistic two-dimensional view of the vessel and lumen profile, as depicted in FIG. 6 and FIG. 7.

Additionally, as depicted in this figure, processor 406 can execute instructions 416 to shade or color the area between vessel border 702 and lumen border 706 to indicate plaque and to shade or color (e.g., in this case add hatch marks) the area between the lumen border 706 and the key frames to indicate the detected stent.

Additionally, as indicated above, processor 406 can be configured to determine an expansion amount (e.g., percentage, ratio, distance, or the like) for the detected stent and display the determined expansion amount in interactive vessel navigation 506. For example, profile view 700 depicts distal bracket ends 610 and 612, which correspond to the proximal and distal key frames, respectively. Further, profile view 700 depicts expansion amounts 714a and 714b, which correspond to the amount the distal and proximal ends of the detected stent, respectively, are expanded. With some embodiments, processor 406 can execute instructions 416 to determine the location of the distal bracket ends 610 and 612 based on the distal and proximal ends of the stent. For example, processor 406 can execute instructions 416 to place the key frames a specified distance beyond the ends of the stent (e.g., 1 millimeter (mm), 2 mm, between 2 and 6 mm, or the like). Further, processor 406 can execute instructions 416 to determine the stent expansion based on the minimum stent area (MSA) divided by the lumen area multiplied by 100.

The interactive vessel navigation 506 is interactive and/or manipulatable by a user. For example, a user can move (e., via I/O devices 410, or the like) the location of the distal bracket end 610 and/or proximal bracket end 612. Accordingly, responsive to receiving an indication to change a location of one or both the distal bracket ends 610 and/or 612, processor 406 can execute instructions 416 to regenerate graphical information elements 420 and GUI 422 to represent the updated location of the key frames. Further, processor 406 can execute instructions 416 to determine stent expansion based on the updated location of the key frames and display the updated stent expansion amounts in GUI 422.

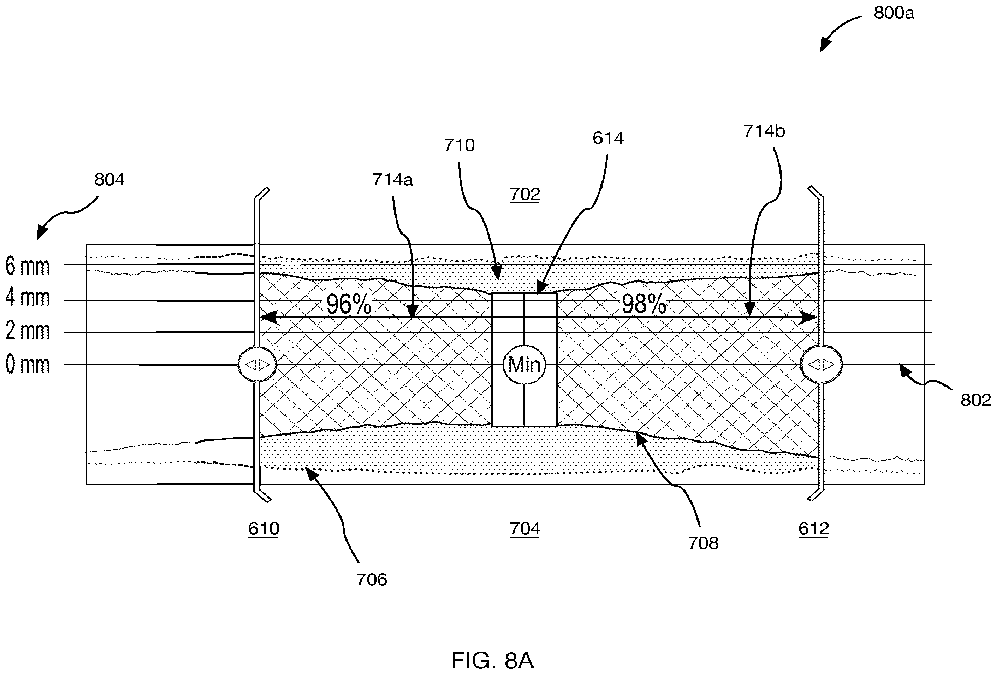

FIG. 8A illustrates profile view 800a, according to some embodiments of the present disclosure. IVUS images visualization system 400 can be configured to generate profile view 800a as interactive vessel navigation 506. As depicted, profile view 800a includes several GUI components including a central axis 802 about which longitudinal border profile 704 and longitudinal border profile mirror reflection 708 are disposed.

Examples of longitudinal border profile 704 and 708 are given above. For example, 704 depicts or is representative of the detected borders (e.g., vessel border 702, lumen border 706, etc.) for the IVUS images 418. Longitudinal border profile mirror reflection 708 is a mirror reflection of the longitudinal border profile 704, thereby providing a more complete visualization of the vessel and lumen profile along with the deployed stent 804

Profile view 800a further includes scale 806 depicting the radius of the detected borders represented in longitudinal border profile 704. Additionally, profile view 800a includes distal bracket end 610 and proximal bracket end 612 as well as minimum region 614. Each of the brackets are movable via user input (e.g., via I/O devices 410). Furthermore, as noted, the amount of expansion of the stent 804 is depicted in both the distal and proximal directions from the minimum region 614 via expansion indicator 808 and expansion indicator 810.

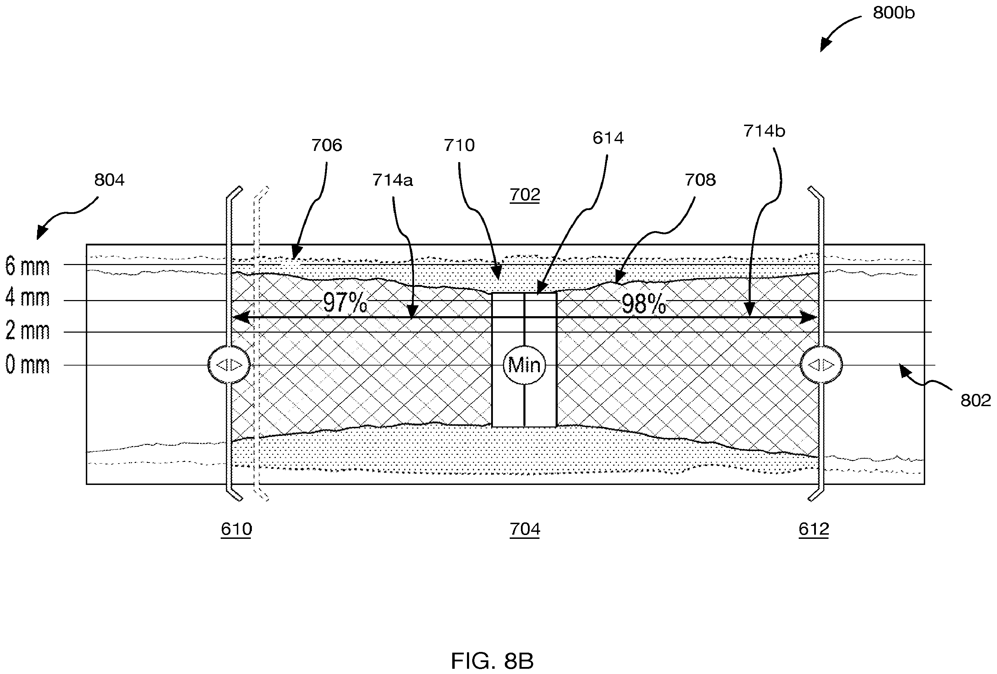

FIG. 8B illustrates profile view 800b, according to some embodiments of the present disclosure. IVUS images visualization system 400 can be configured to generate profile view 800b as interactive vessel navigation 506 responsive to a change in one or both the 610 and/or proximal bracket end 612. For example, a user can move (e., via I/O devices 410, or the like) the location of the distal bracket end 610 and/or proximal bracket end 612. FIG. 8B illustrates the location of 610 depicted in profile view 800b moved with respect to the location of distal bracket end 610 depicted profile view 800a of FIG. 8A. As outlined herein, responsive to a modification of the location of distal bracket end 610 and/or proximal bracket end 612 processor 406 can execute instructions 416 to determine an updated amount of expansion and regenerate graphical information elements 420 and GUI 422 to indicate the stent 804 filling the new space between distal bracket end 610 and proximal bracket end 612 as well as the indications of stent expansion (e.g., expansion indicator 808 and expansion indicator 810).

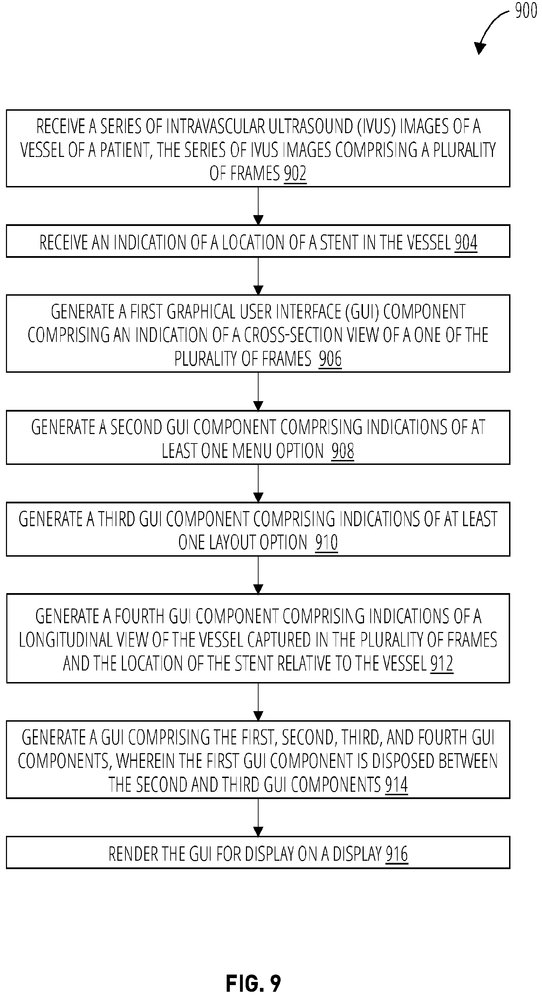

FIG. 9 illustrates a logic flow 900 to generate a GUI, according to some embodiments of the present disclosure. The logic flow 900 can be implemented by IVUS images visualization system 400 and will be described with reference to IVUS images visualization system 400 for clarity of presentation. However, it is noted that logic flow 900 could also be implemented by an IVUS guidance system different than IVUS images visualization system 400.

Logic flow 900 can begin at block 902. At block 902 "receive a series of intravascular ultrasound (IVUS) images of a vessel of a patient, the series of IVUS images comprising a plurality of frames" a series of IVUS images captured via an IVUS catheter percutaneously inserted in a vessel of a patent can be received. For example, information elements comprising indications of IVUS images 418 can be received from IVUS imaging system 100 where catheter 102 is (or was) percutaneously inserted into vessel 202. The IVUS images 418 can comprise frames of images representative of images captured while the catheter 102 is pulled back from distal end 204 to proximal end 206. Processor 406 can execute instructions 416 to receive information elements comprising indications of IVUS images 418 from IVUS imaging system 100, or directly from catheter 102 as may be the case.

Continuing to block 904 "receive an indication of a location of a stent in the vessel" an indication of a location of a stent in the vessel captured in the IVUS images can be received. In some examples, an indication of the frames where the stent is identified are received. For example, processor 406 can execute instructions 416 to receive an indication of the frames in IVUS images 418 where the stent 804 is identified or present. In some examples, processor 406 can execute instructions 416 to detect the stent based on a machine learning model (e.g., an image classification model, or the like).

Continuing to block 906 "generate a first graphical user interface (GUI) component comprising an indication of a cross-section view of a one of the plurality of frames" a first GUI component comprising an indication of a cross-section view of a one of the plurality of frames is generated. For example, processor 406 can execute instructions 416 to generate a cross-section view (e.g., cross-section view 602, or the like).

Continuing to block 908 "generate a second GUI component comprising indications of at least one menu option" a second GUI component comprising indications of at least one menu option is generated. For example, processor 406 can execute instructions 416 to generate menu 502a. Continuing to block 910 "generate a third GUI component comprising indications of at least one layout option" a third GUI component comprising indications of at least one layout option is generated. For example, processor 406 can execute instructions 416 to generate menu 502b. Continuing to block 912 "generate a fourth GUI component comprising indications of a longitudinal view of the vessel captured in the plurality of frames and the location of the stent relative to the vessel" a fourth GUI component comprising indications of a longitudinal view of the vessel represented in the IVUS images and the stent is generated. For example, processor 406 can execute instructions 416 to generate interactive cross-section view 504.

Continuing to block 914 "generate a GUI comprising the first, second, third, and fourth GUI components, wherein the first GUI component is disposed between the second and third GUI components" a GUI comprising the first, second, third, and fourth GUI components where the first GUI component is disposed between the second and third GUI components is generated. For example, processor 406 can execute instructions 416 to generate GUI 422. Continuing to block 916 "render the GUI for display on a display" the GUI can be rendered for display. For example, processor 406 can execute instructions 416 to render the GUI components and GUI for display on display 404.

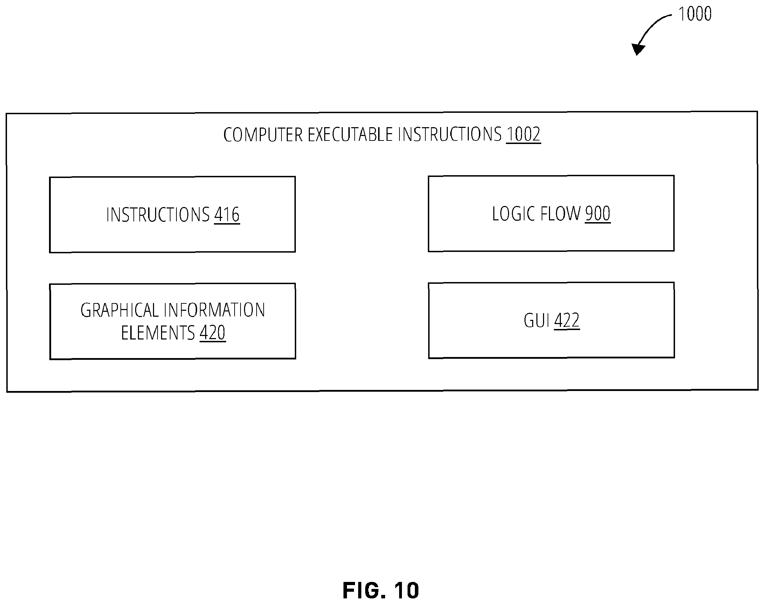

FIG. 10 illustrates computer-readable storage medium 1000. Computer-readable storage medium 1000 may comprise any non-transitory computer-readable storage medium or machine-readable storage medium, such as an optical, magnetic or semiconductor storage medium. In various embodiments, computer-readable storage medium 1000 may comprise an article of manufacture. In some embodiments, computer-readable storage medium 1000 may store computer executable instructions 1002 with which circuitry (e.g., processor 106, processor 406, IVUS imaging system acquisition circuitry 414, and the like) can execute. For example, computer executable instructions 1002 can include instructions to implement operations described with respect to instructions 416, logic flow 900, graphical information elements 420, and/or GUI 422. Examples of computer-readable storage medium 1000 or machine-readable storage medium may include any tangible media capable of storing electronic data, including volatile memory or non-volatile memory, removable or non-removable memory, erasable or non-erasable memory, writeable or re-writeable memory, and so forth. Examples of computer executable instructions 1002 may include any suitable type of code, such as source code, compiled code, interpreted code, executable code, static code, dynamic code, object-oriented code, visual code, and the like.

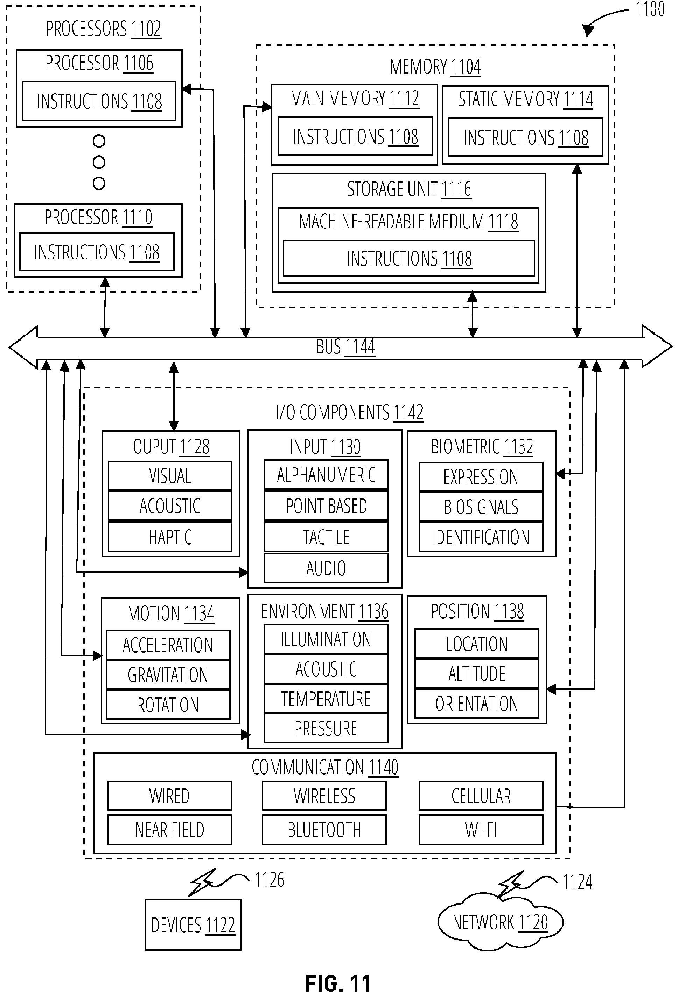

FIG. 11 illustrates a diagrammatic representation of a machine 1100 in the form of a computer system within which a set of instructions may be executed for causing the machine to perform any one or more of the methodologies discussed herein. More specifically, FIG. 11 shows a diagrammatic representation of the machine 1100 in the example form of a computer system, within which instructions 1108 (e.g., software, a program, an application, an applet, an app, or other executable code) for causing the machine 1100 to perform any one or more of the methodologies discussed herein may be executed. For example, the instructions 1108 may cause the machine 1100 to execute logic flow 900 of FIG. 9, instructions 416 of FIG. 4. More generally, the instructions 1108 may cause the machine 1100 to generate GUIs with functionality and behavior as described herein during a pre-PCI, peri-PCI, or post-PCT using IVUS. It is noted that the present disclosure provides specific and discrete implementations of GUI representations and behavior that is a significant improvement over the prior art. In particular, the present disclosure provides an improvement to computing technology in that GUIs provide greater visibility and navigation of IVUS images.

The instructions 1108 transform the general, non-programmed machine 1100 into a particular machine 1100 programmed to carry out the described and illustrated functions in a specific manner. In alternative embodiments, the machine 1100 operates as a standalone device or may be coupled (e.g., networked) to other machines. In a networked deployment, the machine 1100 may operate in the capacity of a server machine or a client machine in a server-client network environment, or as a peer machine in a peer-to-peer (or distributed) network environment. The machine 1100 may comprise, but not be limited to, a server computer, a client computer, a personal computer (PC), a tablet computer, a laptop computer, a netbook, a set-top box (STB), a PDA, an entertainment media system, a cellular telephone, a smart phone, a mobile device, a wearable device (e.g., a smart watch), a smart home device (e.g., a smart appliance), other smart devices, a web appliance, a network router, a network switch, a network bridge, or any machine capable of executing the instructions 1108, sequentially or otherwise, that specify actions to be taken by the machine 1100. Further, while only a single machine 1100 is illustrated, the term "machine" shall also be taken to include a collection of machines 1100 that individually or jointly execute the instructions 1108 to perform any one or more of the methodologies discussed herein.

The machine 1100 may include processors 1102, memory 1104, and I/O components 1142, which may be configured to communicate with each other such as via a bus 1144. In an example embodiment, the processors 1102 (e.g., a Central Processing Unit (CPU), a Reduced Instruction Set Computing (RISC) processor, a Complex Instruction Set Computing (CISC) processor, a Graphics Processing Unit (GPU), a Digital Signal Processor (DSP), an ASIC, a Radio-Frequency Integrated Circuit (RFIC), another processor, or any suitable combination thereof) may include, for example, a processor 1106 and a processor 1110 that may execute the instructions 1108. The term "processor" is intended to include multi-core processors that may comprisc two or more independent processors (sometimes referred to as "cores") that may execute instructions contemporaneously. Although FIG. 11 shows multiple processors 1102, the machine 1100 may include a single processor with a single core, a single processor with multiple cores (e.g., a multi-core processor), multiple processors with a single core, multiple processors with multiples cores, or any combination thereof.

The memory 1104 may include a main memory 1112, a static memory 1114, and a storage unit 1116, both accessible to the processors 1102 such as via the bus 1144. The main memory 1104, the static memory 1114, and storage unit 1116 store the instructions 1108 embodying any one or more of the methodologies or functions described herein. The instructions 1108 may also reside, completely or partially, within the main memory 1112, within the static memory 1114, within machine-readable medium 1118 within the storage unit 1116, within at least one of the processors 1102 (e.g., within the processor's cache memory), or any suitable combination thereof, during execution thereof by the machine 1100.

The I/O components 1142 may include a wide variety of components to receive input, provide output, produce output, transmit information, exchange information, capture measurements, and so on. The specific I/O components 1142 that are included in a particular machine will depend on the type of machine. For example, portable machines such as mobile phones will likely include a touch input device or other such input mechanisms. while a headless server machine will likely not include such a touch input device. It will be appreciated that the I/O components 1142 may include many other components that are not shown in FIG. 11. The I/O components 1142 are grouped according to functionality merely for simplifying the following discussion and the grouping is in no way limiting. In various example embodiments, the I/O components 1142 may include output components 1128 and input components 1130. The output components 1128 may include visual components (e.g., a display such as a plasma display panel (PDP), a light emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components 1130 may include alphanumeric input components (e.g., a keyboard, a touch screen configured to receive alphanumeric input, a photo-optical keyboard, or other alphanumeric input components), point-based input components (e.g., a mouse, a touchpad, a trackball, a joystick, a motion sensor, or another pointing instrument), tactile input components (e.g., a physical button, a touch screen that provides location and/or force of touches or touch gestures, or other tactile input components), audio input components (e.g., a microphone), and the like.

In further example embodiments, the I/O components 1142 may include biometric components 1132, motion components 1134, environmental components 1136, or position components 1138, among a wide array of other components. For example, the biometric components 1132 may include components to detect expressions (e.g., hand expressions, facial expressions, vocal expressions, body gestures, or eye tracking), measure biosignals (e.g., blood pressure, heart rate, body temperature, perspiration, or brain waves), identify a person (e.g., voice identification, retinal identification, facial identification, fingerprint identification, or electroencephalogram-based identification), and the like. The motion components 1134 may include acceleration sensor components (e.g., accelerometer), gravitation sensor components, rotation sensor components (e.g., gyroscope), and so forth. The environmental components 1136 may include, for example, illumination sensor components (e.g., photometer), temperature sensor components (e.g., one or more thermometers that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment. The position components 1138 may include location sensor components (e.g., a GPS receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 1142 may include communication components 1140 operable to couple the machine 1100 to a network 1120 or devices 1122 via a coupling 1124 and a coupling 1126, respectively. For example, the communication components 1140 may include a network interface component or another suitable device to interface with the network 1120. In further examples, the communication components 1140 may include wired communication components, wireless communication components, cellular communication components, Near Field Communication (NFC) components, Bluetooth^{®} components (e.g., Bluetooth^{®} Low Energy), Wi-Fi^{®} components, and other communication components to provide communication via other modalities. The devices 1122 may be another machine or any of a wide variety of peripheral devices (e.g., a peripheral device coupled via a USB).

Moreover, the communication components 1140 may detect identifiers or include components operable to detect identifiers. For example, the communication components 1140 may include Radio Frequency Identification (RFID) tag reader components, NFC smart tag detection components, optical reader components (e.g., an optical sensor to detect one-dimensional bar codes such as Universal Product Code (UPC) bar code, multi-dimensional bar codes such as Quick Response (QR) code, Aztec code, Data Matrix, Dataglyph, MaxiCode, PDF417, Ultra Code, UCC RSS-2D bar code, and other optical codes), or acoustic detection components (e.g., microphones to identify tagged audio signals). In addition, a variety of information may be derived via the communication components 1140, such as location via Internet Protocol (IP) geolocation, location via Wi-Fi^{®} signal triangulation, location via detecting an NFC beacon signal that may indicate a particular location, and so forth.

The various memories (i.e., memory 1104, main memory 1112, static memory 1114, and/or memory of the processors 1102) and/or storage unit 1116 may store one or more sets of instructions and data structures (e.g., software) embodying or utilized by any one or more of the methodologies or functions described herein. These instructions (e.g., the instructions 1108), when executed by processors 1102, cause various operations to implement the disclosed embodiments.

As used herein, the terms "machine-storage medium," "device-storage medium," "computer-storage medium" mean the same thing and may be used interchangeably in this disclosure. The terms refer to a single or multiple storage devices and/or media (e.g., a centralized or distributed database, and/or associated caches and servers) that store executable instructions and/or data. The terms shall accordingly be taken to include, but not be limited to, solid-state memories, and optical and magnetic media, including memory internal or external to processors. Specific examples of machine-storage media, computer-storage media and/or device-storage media include non-volatile memory, including by way of example semiconductor memory devices, e.g., erasable programmable read-only memory (EPROM), electrically erasable programmable read-only memory (EEPROM), FPGA, and flash memory devices; magnetic disks such as internal hard disks and removable disks; magneto-optical disks; and CD-ROM and DVD-ROM disks. The terms "machine-storage media," "computer-storage media," and "device-storage media" specifically exclude carrier waves, modulated data signals, and other such media, at least some of which are covered under the term "signal medium" discussed below.

In various example embodiments, one or more portions of the network 1120 may be an ad hoc network, an intranet, an extranet, a VPN, a LAN, a WLAN, a WAN, a WWAN, a MAN, the Internet, a portion of the Internet, a portion of the PSTN, a plain old telephone service (POTS) network, a cellular telephone network, a wireless network, a Wi-Fi^{®} network, another type of network, or a combination of two or more such networks. For example, the network 1120 or a portion of the network 1120 may include a wireless or cellular network, and the coupling 1124 may be a Code Division Multiple Access (CDMA) connection, a Global System for Mobile communications (GSM) connection, or another type of cellular or wireless coupling. In this example, the coupling 1124 may implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High Speed Packet Access (HSPA), Worldwide Interoperability for Microwave Access (WiMAX), Long Term Evolution (LTE) standard, others defined by various standard-setting organizations, other long range protocols, or other data transfer technology.

The instructions 1108 may be transmitted or received over the network 1120 using a transmission medium via a network interface device (e.g., a network interface component included in the communication components 1140) and utilizing any one of several well-known transfer protocols (e.g., hypertext transfer protocol (HTTP)). Similarly, the instructions 1108 may be transmitted or received using a transmission medium via the coupling 1126 (e.g., a peer-to-peer coupling) to the devices 1122. The terms "transmission medium" and "signal medium" mean the same thing and may be used interchangeably in this disclosure. The terms "transmission medium" and "signal medium" shall be taken to include any intangible medium that can store, encoding, or carrying the instructions 1108 for execution by the machine 1100, and includes digital or analog communications signals or other intangible media to facilitate communication of such software. Hence, the terms "transmission medium" and "signal medium" shall be taken to include any form of modulated data signal, carrier wave, and so forth. The term "modulated data signal" means a signal that has one or more of its characteristics set or changed in such a matter as to encode information in the signal.

Terms used herein should be accorded their ordinary meaning in the relevant arts, or the meaning indicated by their use in context, but if an express definition is provided, that meaning controls.

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to one or multiple ones. Additionally, the words "herein," "above," "below" and words of similar import, when used in this application, refer to this application as a whole and not to any portions of this application. When the claims use the word "or" in reference to a list of two or more items, that word covers all the following interpretations of the word: any of the items in the list, all the items in the list and any combination of the items in the list, unless expressly limited to one or the other. Any terms not expressly defined herein have their conventional meaning as commonly understood by those having skill in the relevant art(s).

By using genuine models of anatomy more accurate surgical plans may be developed than through statistical modeling.

Terms used herein should be accorded their ordinary meaning in the relevant arts, or the meaning indicated by their use in context, but if an express definition is provided, that meaning controls.

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to one or multiple ones. Additionally, the words "herein," "above," "below" and words of similar import, when used in this application, refer to this application as a whole and not to any portions of this application. When the claims use the word "or" in reference to a list of two or more items, that word covers all the following interpretations of the word: any of the items in the list, all the items in the list and any combination of the items in the list, unless expressly limited to one or the other. Any terms not expressly defined herein have their conventional meaning as commonly understood by those having skill in the relevant art(s).

## Claims

1. A method, comprising:
receiving a series of intravascular ultrasound, IVUS, images of a vessel of a patient, the series of IVUS images comprising a plurality of frames;
receiving an indication of a location of a stent (804) in the vessel;
generating a first graphical user interface, GUI, component comprising an indication of a cross-section view of the vessel captured in one of the plurality of frames;
generating a second GUI component comprising indications of at least one menu option;
generating a third GUI component comprising indications of at least one layout option;
generating a fourth GUI component comprising indications of a longitudinal view of the vessel captured in the plurality of frames and the location of the stent (804) relative to the vessel;
determining a distal expansion of the stent (804) and a proximal expansion of the stent (804), wherein the fourth GUI component comprises an indication of the distal expansion of the stent (804) and an indication of the proximal expansion of the stent (804);
generating a GUI comprising the first, second, third, and fourth GUI components, wherein the first GUI component is disposed between the second and third GUI components; and
rendering the GUI for display on a display (404).

2. The method of claim 1, comprising:
generating a proximal key frame based on a proximal end of the stent (804);
generating a distal key frame based on a distal end of the stent (804); and
generating the fourth GUI component further comprising an indication of the proximal key frame and the distal key frame.

3. The method of claim 2, wherein the fourth GUI component comprises an indication of the vessel and an indication of a lumen.

4. The method of claim 3, wherein the fourth GUI component comprises a mirrored reflection of the indication of the vessel and the lumen about a longitudinal axis.

5. The method of claim 4, wherein the fourth GUI component comprises a colored, shaded, or patterned area between the lumen and the mirrored reflection of the lumen and the proximal key frame and the distal key frame to indicate the location of the stent (804) relative to the vessel.

6. The method of claim 5, comprising determining an expansion of the stent (804), wherein the fourth GUI component comprises an indication of the expansion of the stent (804).

7. The method of any of the preceding claims, comprising:
determining the distal expansion of the stent (804) based on a minimum stent area, MSA, divided by a lumen area for areas distal of a minimum key frame; and
determining the proximal expansion of the stent (804) based on the minimum stent area, MSA, divided by the lumen area for areas proximal of the minimum key frame.

8. The method of claim 7, wherein the distal expansion of the stent (804) and the proximal expansion of the stent (804) are represented as percent, wherein the indication of the distal expansion of the stent (804) comprises a line between the minimum frame marker and a distal end of the stent (804) and a graphical indication of the percent of distal expansion, and wherein the indication of the proximal expansion of the stent (804) comprises a line between the minimum frame marker and a proximal end of the stent (804) and a graphical indication of the percent of proximal expansion.

9. The method of claim 8, comprising receiving, via an input device, an indication to move the proximal key frame, the distal key frame, or the proximal key frame and the distal key frame.

10. The method of claim 9, comprising determining an updated distal expansion of the stent (804) and/or an updated proximal expansion of the stent (804) based on the moved proximal key frame and/or the moved distal key frame.

11. The method of claim 10, comprising regenerating the fourth GUI component and the GUI to include an indication of the updated distal expansion of the stent (804) and/or the updated proximal expansion of the stent (804).

12. The method of claim 11, wherein the regenerated fourth GUI components comprises the colored, shaded, or patterned area between the lumen and the mirrored reflection of the lumen and the moved proximal key frame and/or the moved distal key frame to indicate the updated location of the stent (804) relative to the vessel.

13. An apparatus, comprising a processor (106; 406; 1102) coupled to a memory (408; 1104), the memory (408; 1104) comprising instructions executable by the processor (106; 406; 1102), the processor (106; 406; 1102) configured to couple to an intravascular ultrasound, IVUS, imaging system (100; 414) and configured to execute the instructions, which instructions when executed cause the processor (106; 406; 1102) to implement the method of any one of claims 1 to 12.

14. At least one machine readable storage device, comprising a plurality of instructions that in response to being executed by a processor (106; 406; 1102) of an intravascular ultrasound, IVUS, imaging system (100; 414) cause the processor (106; 406; 1102) to implement the method of any one of claims 1 to 12.

## Patentansprüche

1. Verfahren, aufweisend:
Empfangen einer Reihe von intravaskulären Ultraschallbildern, IVUS, eines Gefäßes eines Patienten, wobei die Reihe von IVUS-Bildern mehrere Frames aufweist;
Empfangen einer Indikation einer Position eines Stents (804) in dem Gefäß;
Erzeugen einer ersten grafischen Benutzeroberflächen-, GUI, Komponente, die eine Indikation für eine Querschnittsansicht des Gefäßes aufweist, die in einem der mehreren Frames erfasst wurde;
Erzeugen einer zweiten GUI-Komponente, die Indikationen mindestens einer Menüoption aufweist;
Erzeugen einer dritten GUI-Komponente, die Indikationen mindestens einer Layout-Option aufweist;
Erzeugen einer vierten GUI-Komponente, die Indikationen einer Längsansicht des Gefäßes, das in den mehreren Frames erfasst ist, und der Position des Stents (804) relativ zum Gefäß aufweist;
Bestimmen einer distalen Expansion des Stents (804) und einer proximalen Expansion des Stents (804), wobei die vierte GUI-Komponente eine Indikation der distalen Expansion des Stents (804) und eine Indikation der proximalen Expansion des Stents (804) aufweist;
Erzeugen einer GUI, die die erste, zweite, dritte und vierte GUI-Komponente aufweist, wobei die erste GUI-Komponente zwischen der zweiten und dritten GUI-Komponente angeordnet ist; und
Rendern der GUI zur Anzeige auf einer Anzeige (404).

2. Verfahren nach Anspruch 1, aufweisend:
Erzeugen eines proximalen Keyframes basierend auf einem proximalen Ende des Stents (804);
Erzeugen eines distalen Keyframes basierend auf einem distalen Ende des Stents (804); und
Erzeugen der vierten GUI-Komponente, die ferner eine Indikation des proximalen Keyframes und des distalen Keyframes aufweist.

3. Verfahren nach Anspruch 2, wobei die vierte GUI-Komponente eine Indikation des Gefäßes und eine Indikation eines Lumens aufweist.

4. Verfahren nach Anspruch 3, wobei die vierte GUI-Komponente eine gespiegelte Reflexion der Indikation des Gefäßes und des Lumens um eine Längsachse aufweist.

5. Verfahren nach Anspruch 4, wobei die vierte GUI-Komponente einen farbigen, schattierten oder gemusterten Bereich zwischen dem Lumen und der gespiegelten Reflexion des Lumens und dem proximalen Keyframe und dem distalen Keyframe aufweist, um die Position des Stents (804) relativ zum Gefäß anzuzeigen.

6. Verfahren nach Anspruch 5, das das Bestimmen einer Expansion des Stents (804) aufweist, wobei die vierte GUI-Komponente eine Indikation der Expansion des Stents (804) aufweist.

7. Verfahren nach einem der vorhergehenden Ansprüche, das aufweist:
Bestimmen der distalen Expansion des Stents (804) basierend auf einer minimalen Stentfläche, MSA, geteilt durch eine Lumenfläche für Bereiche distal eines minimalen Keyframes; und
Bestimmen der proximalen Expansion des Stents (804) basierend auf der minimalen Stentfläche, MSA, geteilt durch die Lumenfläche für Bereiche proximal des minimalen Keyframes.

8. Verfahren nach Anspruch 7, wobei die distale Expansion des Stents (804) und die proximale Expansion des Stents (804) in Prozent dargestellt werden, wobei die Indikation der distalen Expansion des Stents (804) eine Linie zwischen der minimalen Frame-Markierung und einem distalen Ende des Stents (804) und eine grafische Indikation des Prozentsatzes der distalen Expansion aufweist, und wobei die Indikation der proximalen Expansion des Stents (804) eine Linie zwischen der minimalen Frame-Markierung und einem proximalen Ende des Stents (804) und eine grafische Indikation des Prozentsatzes der proximalen Expansion aufweist.

9. Verfahren nach Anspruch 8, das das Empfangen über eine Eingabevorrichtung einer Indikation aufweist, um den proximalen Keyframe, den distalen Keyframe oder den proximalen Keyframe und den distalen Keyframe zu bewegen.

10. Verfahren nach Anspruch 9, das das Bestimmen einer aktualisierten distalen Expansion des Stents (804) und/oder einer aktualisierten proximalen Expansion des Stents (804) basierend auf dem bewegten proximalen Keyframe und/oder dem bewegten distalen Keyframe aufweist.

11. Verfahren nach Anspruch 10, das das Regenerieren der vierten GUI-Komponente und der GUI aufweist, um eine Indikation der aktualisierten distalen Expansion des Stents (804) und/oder der aktualisierten proximalen Expansion des Stents (804) zu enthalten.

12. Verfahren nach Anspruch 11, wobei die regenerierte vierte GUI-Komponente den gefärbten, schattierten oder gemusterten Bereich zwischen dem Lumen und der gespiegelten Reflexion des Lumens und dem bewegten proximalen Keyframe und/oder dem bewegten distalen Keyframe aufweist, um die aktualisierte Position des Stents (804) relativ zu dem Gefäß anzuzeigen.

13. Vorrichtung, die einen Prozessor (106; 406; 1102) aufweist, der mit einem Speicher (408; 1104) gekoppelt ist, wobei der Speicher (408; 1104) von dem Prozessor (106; 406; 1102) ausführbare Anweisungen aufweist, wobei der Prozessor (106; 406; 1102) konfiguriert ist, mit einem intravaskulären Ultraschall-, IVUS-, Bildgebungssystem (100; 414) gekoppelt zu werden und konfiguriert ist, die Anweisungen auszuführen, wobei die Anweisungen, wenn sie ausgeführt werden, den Prozessor (106; 406; 1102) veranlassen, das Verfahren nach einem der Ansprüche 1 bis 12 zu implementieren.

14. Mindestens eine maschinenlesbare Speichervorrichtung, die mehrere Anweisungen aufweist, die als Reaktion darauf, dass sie durch einen Prozessor (106; 406; 1102) eines intravaskulären Ultraschall-, IVUS-, Bildgebungssystems (100; 414) ausgeführt werden, den Prozessor (106; 406; 1102) veranlassen, das Verfahren nach einem der Ansprüche 1 bis 12 zu implementieren.

## Revendications

1. Procédé, comprenant :
la réception d'une série d'images ultrasonores intravasculaires, IVUS, d'un vaisseau d'un patient, la série d'images IVUS comprenant une pluralité de trames ;
la réception d'une indication d'un emplacement d'un stent (804) dans le vaisseau ;
la génération d'un premier composant d'interface graphique utilisateur, GUI, comprenant une indication d'une vue en coupe transversale du vaisseau capturé dans l'une de la pluralité de trames ;
la génération d'un deuxième composant de GUI comprenant des indications d'au moins une option de menu ;
la génération d'un troisième composant de GUI comprenant des indications d'au moins une option de mise en page ;
la génération d'un quatrième composant de GUI comprenant des indications d'une vue longitudinale du vaisseau capturé dans la pluralité de trames et l'emplacement du stent (804) par rapport au vaisseau ;
la détermination d'une expansion distale du stent (804) et d'une expansion proximale du stent (804), dans lequel le quatrième composant de GUI comprend une indication de l'expansion distale du stent (804) et une indication de l'expansion proximale du stent (804) ;
la génération d'une GUI comprenant les premier, deuxième, troisième et quatrième composants de GUI, dans lequel le premier composant de GUI est disposé entre les deuxième et troisième composants de GUI ; et
rendre la GUI pour l'afficher sur un affichage (404).

2. Procédé selon la revendication 1, comprenant :
la génération d'une trame clé proximale sur la base d'une extrémité proximale du stent (804) ;
la génération d'une trame clé distale sur la base d'une extrémité distale du stent (804) ; et
la génération du quatrième composant de GUI comprenant en outre une indication de la trame clé proximale et de la trame clé distale.

3. Procédé selon la revendication 2, dans lequel le quatrième composant de GUI comprend une indication du vaisseau et une indication d'une lumière.

4. Procédé selon la revendication 3, dans lequel le quatrième composant de GUI comprend une réflexion symétrique de l'indication du vaisseau et de la lumière par rapport à un axe longitudinal.

5. Procédé selon la revendication 4, dans lequel le quatrième composant de GUI comprend une zone colorée, ombrée ou à motifs entre la lumière et la réflexion symétrique de la lumière et la trame clé proximale et la trame clé distale pour indiquer l'emplacement du stent (804) par rapport au vaisseau.

6. Procédé selon la revendication 5, comprenant la détermination d'une expansion du stent (804), dans lequel le quatrième composant de GUI comprend une indication de l'expansion du stent (804).

7. Procédé selon l'une des revendications précédentes, comprenant :
la détermination de l'expansion distale du stent (804) sur la base d'une zone de stent minimale, MSA, divisée par une zone de lumière pour les zones distales d'une trame clé minimale ; et
la détermination de l'expansion proximale du stent (804) sur la base de la zone de stent minimale, MSA, divisée par la zone de lumière pour les zones proximales de la trame clé minimale.

8. Procédé selon la revendication 7, dans lequel l'expansion distale du stent (804) et l'expansion proximale du stent (804) sont représentées en pourcentage, dans lequel l'indication de l'expansion distale du stent (804) comprend une ligne entre le marqueur de trame minimale et une extrémité distale du stent (804) et une indication graphique du pourcentage d'une expansion distale, et dans lequel l'indication de l'expansion proximale du stent (804) comprend une ligne entre le marqueur de trame minimale et une extrémité proximale du stent (804) et une indication graphique du pourcentage d'une expansion proximale.

9. Procédé selon la revendication 8, comprenant la réception via un dispositif d'entrée d'une indication de déplacer la trame clé proximale, la trame clé distale, ou la trame clé proximale et la trame clé distale.

10. Procédé selon la revendication 9, comprenant la détermination d'une expansion distale mise à jour du stent (804) et/ou d'une expansion proximale mise à jour du stent (804) sur la base de la trame clé proximale déplacée et/ou de la trame clé distale déplacée.

11. Procédé selon la revendication 10, comprenant la régénération du quatrième composant de GUI et de la GUI afin d'y inclure une indication de l'expansion distale mise à jour du stent (804) et/ou de l'expansion proximale mise à jour du stent (804).

12. Procédé selon la revendication 11, dans lequel les quatrièmes composants de GUI régénérés comprennent la zone colorée, ombrée ou à motifs entre la lumière et la réflexion symétrique de la lumière et la trame clé proximale déplacée et/ou la trame clé distale déplacée pour indiquer l'emplacement mis à jour du stent (804) par rapport au vaisseau.

13. Appareil, comprenant un processeur (106; 406 ; 1102) couplé à une mémoire (408 ; 1104), la mémoire (408 ; 1104) comprenant des instructions exécutables par le processeur (106 ; 406 ; 1102), le processeur (106 ; 406 ; 1102) étant configuré pour être couplé à un système d'imagerie ultrasonore intravasculaire, IVUS, (100 ; 414) et configuré pour exécuter les instructions, lesquelles instructions, lorsqu'elles sont exécutées, amènent le processeur (106 ; 406 ; 1102) à mettre en œuvre le procédé selon l'une des revendications 1 à 12.

14. Au moins un dispositif de stockage lisible par machine, comprenant une pluralité d'instructions qui, en réponse à leur exécution par un processeur (106 ; 406 ; 1102) d'un système d'imagerie ultrasonore intravasculaire, IVUS, (100 ; 414), amènent le processeur (106 ; 406 ; 1102) à mettre en œuvre le procédé selon l'une des revendications 1 à 12.
